# EUROPEAN PATENT SPECIFICATION

(11) **EP 2 668 437 B1**
(45) Date of publication and mention of the grant of the patent: **27.05.2020**
(21) Application number: 11729542.8
(22) Date of filing: 06.04.2011
(51) Int. Cl.: F16M 11/10, F16M 11/04, F16M 11/08, F16M 11/24, F16M 13/02, A61B 90/50

(54) **SPATIALLY ADJUSTABLE ARM**
RÄUMLICH EINSTELLBARER ARM
BRAS RÉGLABLE SPATIALEMENT

(30) Priority: 28.01.2011 CZ 201123867 U
(43) Date of publication of application: 04.12.2013
(73) Proprietor: MZ Liberec, A.S., 543 72 Rudník (CZ)
(72) Inventor: VANEK, Václav, 37816 Lomnice nad Luznici (CZ); VANOUCEK, Jan, 51236 Horní Branná (CZ)
(74) Representative: Skoda, Milan
(86) International application number: PCT/CZ2011/000031
(87) International publication number: WO 2012/100756

(56) References cited:
- WO-A1-2004/038276
- CZ-B6- 302 671
- CZ-U1- 21 940
- DE-A1- 19 746 521
- GB-A- 515 650
- US-A- 2 110 561
- US-A- 4 160 536
- US-A1- 2004 188 578
- US-A1- 2005 205 734

## Description

### Sphere of Technology

The invention deals with a spatially adjustable arm, especially a spatially adjustable arm of a roof stand used to carry a useful load as e.g. operation lights, monitors, video cameras, anti-radiation screens, etc.

### Hitherto State of the Art

At present, several structural designs of height balanced arms used for suspension of medical instruments, aids and devices are known.

A height balanced arm containing a piston damper is e.g. described in the German patent no. DE 102006014217. A disadvantage of this arm is its worse mobility.

An adjustable arm with a swivelling joint and an adapter for lighting technology in medicine in described in the utility model no. CZ 1666 U. Between parts fixed to a supporting tube, especially between a supporting ring and a guide a spring mechanism is mounted ensuring balancing of the arm. This system does not contain any stabilizing element which results in impaired functional usability.

Another utility model, CZ 4170 U, deals with a ceiling arm designed for transfer of medical gaseous and electric media to the working area. The main part of the ceiling arm is at least one rotary supporting bushing to which at least one horizontal arm is attached whose end is fitted with at least one rotary bushing to which either a device head is connected or at least one horizontal arm terminated with at least one rotary bushing connected to a device head while the device head contains quick couplings of medical gases, electric sockets, incl. earthing terminals and monitoring pressure and vacuum gauges. A disadvantage of this arm is its limited vertical adjustability.

The utility model No. CZ 15920 U describes a swinging arm with covered gaps consisting of heads connected with at least one pair of parallel draw-bars of equal length where the pair consists of the first draw-bar and the second draw-bar, which are attached to the side plates in a rotary way around the first axes and second axes while the swinging arm has the top part, bottom part and side part. To the first draw-bar the first part of the cover is attached consisting of the first part overlapping the top part of the switching arm and the first side parts partly overlapping the side parts of the swinging arm while to the second draw-bar the second part of the cover is attached that overlaps the bottom part of the swinging arm and partly with its second side parts the side parts of the arm where the first part of the cover and the second part of the cover adjoin the side plates while the gaps between the first side parts and the second side parts are hidden under covering strips adjoining the first side parts and the second side parts with the possibility of mutual movement between the covering strip and the side parts while the ends of the covering strip are designed in the shape of concave areas, with which they adjoin to convex areas with the corresponding shape created at least in a part of the perimeter of lids that cover the side plates of the heads. Disadvantages of this swinging arm are that its structure is more robust and heavier, it consists of a higher number of parts and therefore it is generally more complex and can only be adjusted with a motor.

A swinging arm whose movement is damped with a gas spring is also known from the European patent no. EP 1067419. A disadvantage of this design is that the swinging arm is not easily adjustable.

US 2005/0205734 discloses a spatially adjustable arm according to the features of the preamble of claim 1.

The above-mentioned state of the art illustrates a number of disadvantages of the prior art while impaired functionality of known structural designs appears to be the most significant of them.

The aim of the invention is to design a swinging arm that will enable easy handling and stable spatial anchoring of suspended accessories.

### Principle of the Invention

The above mentioned shortcomings are resolved to a considerable extent and the aims of the invention are fulfilled by a spatially adjustable arm for a ceiling stand for attaching and carrying suspended accessories such which are medical instruments, systems, aids and devices especially operation lights, monitors, video cameras and anti-radiation screens, with an outer joint, and that is connected to a supporting system of the ceiling stand by means of an inner joint, said spatially adjustable arm contains an arm connected to the inner joint in a rotary way consisting of a beam functioning as a cover for the arm at the ends of which an inner head and an outer head are arranged and the arm contains a balancing mechanism, whereas the balancing mechanism is mounted in the inner head and in the outer head and the inner head and the outer head are pressed onto the beam, in accordance with the invention the principle of which is that the balancing mechanism further contains a stabilizer that is connected to the inner joint and outer joint with the use of a pair of pins, said stabilizer being also contained in said beam. The balancing mechanism allows the balancing of the whole spatially adjustable arm, including the carried useful load. The use of the stabilizer secures the position of the vertical axis of the rotary connection element that is part of the outer joint in the vertical position without deflection. The outer joint is connected to the arm by means of a pin in a rotary way. The stabilizer makes it possible for the axes of the inner joint and outer joint to be always parallel after any height readjustment of the arm.

The arm in which the balancing mechanism is installed forms a sight cover at the same time. This arm design fully meets the aims of the invention, namely easy handling and stable spatial anchoring of suspended accessories. Another advantage of this arm design is in its reduced weight, which has a high influence on the required functionality, but it also results in reduced material costs and thus a lower price. A great advantage is that the arm has fewer gaps where dirt may accumulate as such dirt may be difficult to remove from gaps and may potentially pose a health risk. Other advantages are compactness of the self-contained design a reduced number of parts and elimination of a relatively large and complex plastic cover.

The balancing mechanism is mounted in the inner head and in the outer head while in the most convenient embodiment the balancing mechanism contains a rod that is mounted in the inner head in a sliding way by means of a slider and in the outer head in a sliding way by means of an insert while on the rod an adjustable nut is mounted that leans against a compression spring mounted on the rod whose other side leans against the inner head, the slider is mounted on the rod in a fixed way on which it is secured against movement with a counter nut and the slider is connected to a hook by means of a pin and the hook is, by means of another pin, connected to a stone that is mounted in the inner joint by means of a guide and a setting screw. A change of the mutual position of the rotary pin of the arm and the adjustable pin changes the kinematic proportions of the above-mentioned balancing mechanism and subsequently its carrying capacity. The movement of the adjustable stone is caused by turning of the setting screw that passes through the adjustable stone and is mounted on the inner joint with the use of a thread. The stone moves in the direction of the axis of the setting screw and supporting pin. A change of the position of the adjustable stone will change the distance of the point at which the spring force is applied from the tilting axis of the arm, changing the carrying capacity of the arm. The carrying capacity of the arm is changed to a great extent by pre-tensioning of the compression spring by means of the nut that is screwed on the spring rod. At its other end the spring rod is screwed into a slider moving in a bed while it is secured against spontaneous release with a counter nut of the spring rod.

An advantage is that the balancing mechanism ensuring height balancing of the spatially adjustable arm consists of a low number of simple parts. Another advantage is the ease of fine adjustment of the carrying capacity of the spatially adjustable arm while the basic carrying capacity setting is achieved by compression of the spring of the mechanism with the use of the nut.

In the first embodiment the spatially adjustable arm contains an outer joint connected to the arm in a rotary way on which suspended accessories are mounted - especially operation lights, video cameras, monitors, protective barriers against X-rays, etc.

On the surface of the outer head a fender is mounted in a movable way, which is at the same time movably mounted on the inner side of the cover that is attached to the outer joint while the fender is mounted on a pin. The fender is used to cover the openings in the inner as well as outer part of the arm that appear during the movement of the arm on the vertical plane. This unique solution in entirely faultless, allowing to repeat the assembly and disassembly any number of times and is interesting from the aesthetic point of view.

The use of a stabilizer ensures parallelism of the turning axes of the inner joint assembly of the arm and the outer joint assembly of the arm.

The spatially adjustable arm contains an inner joint connected to the arm in a rotary way with the use of a pin and on the surface of the inner head a movably mounted fender, which is also movably mounted on the inner side of the cover that is attached to the inner joint while the fender is mounted on a pin. An advantage of this unique design is simplicity, operation reliability and the possibility to repeat the assembly and disassembly any number of times.

All the above mentioned covering covers openings that appear due to the movement of the spatially adjustable arm along its while working range while individual parts of the covering do not have any visible fixing points.

The spatially adjustable arms of the ceiling stand are used to hold the above mentioned suspended accessories in medical facilities, but may also be used to carry instruments, aids, devices, etc.; their use is convenient in many types of workplaces, e.g. in the industry. The spatially adjustable arms can be adjusted manually.

The stand in accordance with the invention enables easy and smooth handling of suspended accessories and their precise stabilization in the optimum position set by the operator.

### Overview of Figures in the Drawing

The invention will be illustrated in a more detailed way with the use of a drawing where fig. 1 shows a sectional view of the detailed design of the spatially adjustable arm that contains an outer joint on which suspended accessories are mounted, fig. 2 shows a sectional view of the detailed design of the covering of the spatially adjustable arm that contains an outer joint, and fig.3 presents an axonomical view of the overall design of a stand that contains the spatially adjustable arms in accordance with the invention.

### Sample Embodiment of the Invention

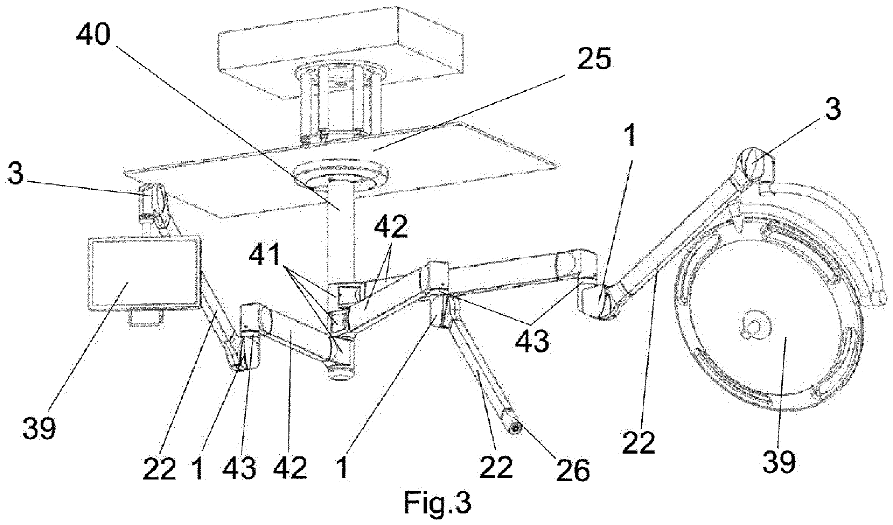

The spatially adjustable arm 22 of the ceiling stand 25 (fig. 3) is used to carry suspended accessories 39 which are medical instruments, aids and devices and is connected to the supporting system of the ceiling stand 25 with an inner joint 1.

The ceiling stand 25 (fig. 3) contains a supporting system 40 to which, with the use of three rotary joints 41, three rotary arms 42 are attached to which the height adjustable parts 43 are fixed to which the spatially adjustable arms 22 are connected to which suspended accessories 39 are fixed. Two of the spatially adjustable arms 22 contain an outer joint 3 on which the suspended accessories 39 are mounted. One of the spatially adjustable arms 22 contains an outer head 26 designed for attachment of suspended accessories 39.

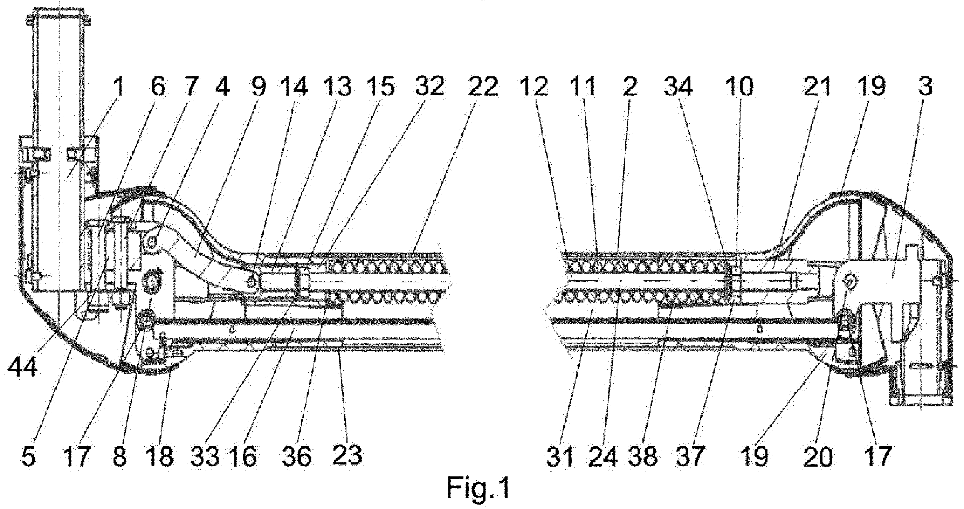

The spatially adjustable arm 22 (fig. 1) contains an arm 23 connected to the inner joint 1 in a rotary way, the arm 23 consisting of a beams 2 at the ends of which the inner head 18 and outer head 19,26 are pressed on and in the arm 23 a height balancing mechanism 24 is arranged. The beam 2 is made of an extruded Al profile. The inner head 18 and outer head 19,26 are made as Al alloy castings.

The height balancing mechanism 24 is mounted in the inner head 18 and in the outer head 19 in a sliding way.

The height adjustable mechanism 24 (fig. 1) contains a rod 12 that is mounted in a sliding way by means of a slider 13 in the inner head 18 and in a sliding way by means of an insert 21 in the outer head 19 while on the rod 12 an adjustable nut 10 is mounted that leans against the compression spring 11 mounted on the rod 12 the other side of which leans against the inner head 18, the slider 13 is fixed to the rod 12, on which it is secured against movement with a counter nut 15, and the slider 13 is, through a pin 14 connected to a hook 9, which is, by means of another pin 4 connected to an adjustable stone 5, which is, by means of a guide 7 and setting screw 6 fitted in the inner joint 1. The height balancing mechanism 24 is mounted in a cavity 31 of the arm 23. The slider 13 is installed in a cavity 32 created in a boss 33 of the inner head 18. The insert 21, mounted in the cavity 37 created in a boss 38 of the outer head 19, is screwed on the rod 12. Between the nut 10 and the compression spring 11 there is a pad 34. The compression spring 11 is seated in a recess 35 of the boss 33 of the inner head 18 while it leans against the pad 36 fitted in the boss 33. The movement of the adjustable stone 5 is caused by turning of the adjustment pin 6, which passes through the adjustable stone 5 while it is mounted in the nut 44 with the use of a thread, the nut being attached to the inner joint 1.

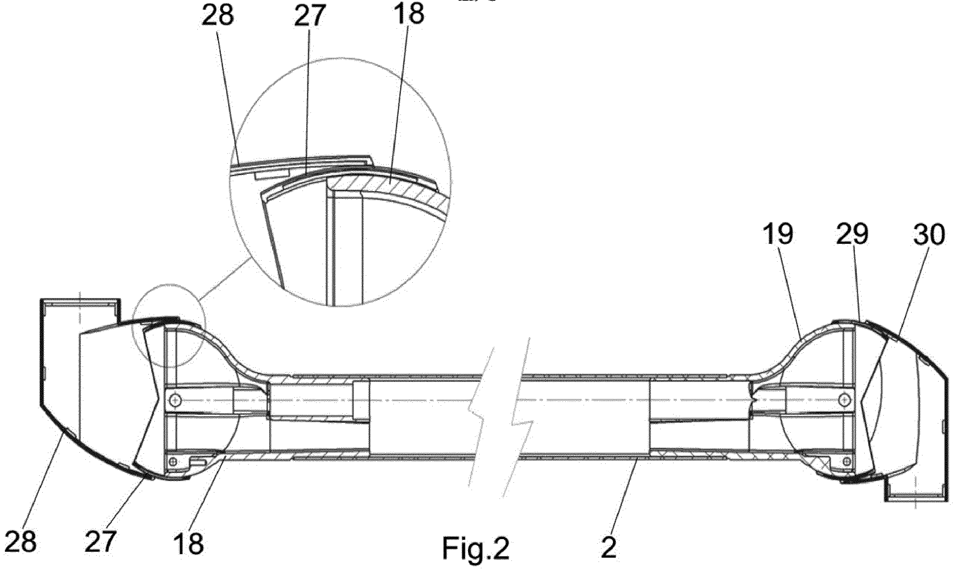

The spatially adjustable arm 22 (fig. 1) further contains an outer joint 3 connected to the arm 23, the joint carrying suspended accessories 39. In this embodiment the spatially adjustable arm 22 contains a balancing mechanism 24 that contains a stabilizer 16 that is connected to the inner joint 1 and to the outer joint 3 with the use of a pair of pins 17 while on the surface of the outer head 19 (fig. 2) a plastic fender 29 is movably mounted, which is also movably mounted on the inner side of the plastic cover 30 attached to the outer joint 3. The plastic fender 29 is mounted on the pin 20 at the same time. The outer joint 3 is connected to the arm 23 in a rotary way by means of a pin 20 seated in the outer head 19 of the arm 23.

The spatially adjustable arm 22 (fig. 2) have a plastic fender 27 that is movably mounted on the surface of the inner head 18 and at the same time movably mounted on the inner side of the plastic cover 28, which is fixed to the inner joint 1 while the plastic fender 27 is mounted on a pin 8, and the spatially adjustable arm 22 the inner joint 1 is connected to the arm 23 in a rotary way by means of a pin 8 seated in the inner head 18 of the arm 23.

### Industrial Utilization

A spatially adjustable arm in accordance with the invention can be used anywhere where various devices, instruments, aids and systems need to be moved in space while its use is mainly convenient in medical facilities.

### List of Reference Marks

- 1: Inner joint
- 2: Beam
- 3: Outer joint
- 4: Pin I
- 5: Stone
- 6: Setting screw
- 7: Guide
- 8: Pin II
- 9: Hook
- 10: Nut I
- 11: Compression spring
- 12: Rod
- 13: Slider
- 14: Pin III
- 15: Counter nut
- 16: Stabilizer
- 17: Pin IV
- 18: Inner head
- 19: Outer head
- 20: Pin V
- 21: Insert
- 22: Height adjustable arm
- 23: Arm
- 24: Balancing mechanism
- 25: Ceiling stand
- 26: Outer head
- 27: Fender I
- 28: Cover I
- 29: Fender II
- 30: Cover II
- 31: Cavity I
- 32: Cavity II
- 33: Boss I
- 34: Pad I
- 35: Recess
- 36: Pad II
- 37: Cavity III
- 38: Boss II
- 39: Suspended accessories
- 40: Supporting system
- 41: Rotary joint
- 42: Rotary arm
- 43: Height adjustable part
- 44: Nut II

## Claims

1. A spatially adjustable arm (22) for a ceiling stand (25) for attaching and carrying suspended accessories (39) which are medical instruments, systems, aids and devices with an outer joint (3), and that is attached with an inner joint (1) to a supporting system of the ceiling stand (25), said spatially adjustable arm (22) contains an arm (23) connected to the inner joint (1) in a rotary way consisting of a beam (2) functioning as a cover for the arm (23) at the ends of which an inner head (18) and an outer head (19,26) are arranged and the arm (23) contains a balancing mechanism (24), whereas the balancing mechanism (24) is mounted in the inner head (18) and in the outer head (19) and the inner head (18) and the outer head (19) are pressed onto the beam (2), said spatially adjustable arm (22) being **characterised in that** the balancing mechanism (24) further contains a stabilizer (16) that is connected to the inner joint (1) and outer joint (3) with the use of a pair of pins (17), said stabilizer (16) being also contained in said beam (2).

2. The spatially adjustable arm accordance with claim 1, **characterised in that** the balancing mechanism (24) contains a rod (12) that is mounted in the inner head (18) in a sliding way by means of a slider (13) and in the outer head (19) in a sliding way by means of an insert (21) while on the rod an adjustable nut (10) is mounted that leans against a compression spring (11) mounted on the rod (12) whose other side leans against the inner head (18), the slider (13) is mounted on the rod (12) in a fixed way on which it is secured against movement with a counter nut (15) and the slider (13) is connected to a hook (9) by means of a pin (14) and the hook (9) is, by means of another pin (4), connected to an adjustable stone (5) that is mounted in the inner joint (1) by means of a guide (7) and a setting screw (6).

3. The spatially adjustable arm accordance with any of the previous claims, **characterised in that** further contained an outer joint (3) connected to the arm (23) in a rotary way the joint (3) being used to carry suspended accessories.

4. The spatially adjustable arm accordance with any of the previous claims, **characterised in that** on the surface of the inner head (18) a fender (27) is movably mounted, which is at the same time movably mounted on the inner side of the cover (28), which is attached to the inner joint while the fender (27) is mounted on a pin (8).

5. The spatially adjustable arm accordance with any of the previous claims, **characterised in that** on the surface of the outer head (19) a fender (29) is movably mounted, which is also movably mounted on the inner side of the cover (30) attached to the outer joint (3) while the fender (29) is mounted on a pin (20).

6. The spatially adjustable arm accordance with any of previous claims, **characterised in that** the outer joint (3) is connected to the arm (23) in a rotary way by means of a pin (20).

7. The spatially adjustable arm accordance with any of the previous claims, **characterised in that** the inner joint (1) is connected to the arm (23) in a rotary way by means of a pin (8).

## Patentansprüche

1. Der räumlich verstellbare Arm (22) des Deckenstativs (25) für das Befestigen und Tragen hängenden Zubehörs (39) wie medizinische Instrumente, Systeme, Hilfsmittel und Geräte mit Außengelenk (3), welcher mittels eines inneren Gelenks (1) an das das Tragsystem des Deckenstativs (25) befestigt wird, wobei der räumlich verstellbare Arm (22) einen drehbar verbundenen Arm (23) mit einem inneren Gelenk (1) enthält, der aus einem Träger (2) besteht, an dessen Enden der innere Kopf (18) und der Außenkopf (19,26) angebracht sind, sowie ein im Arm (23) errichteter Ausgleichsmechanismus (24), während dessen der Ausgleichsmechanismus (24) im inneren Kopf (18) und im Außenkopf (19) eingebracht wird, und der innere Kopf (18) und der Außenkopf (19) sind auf den Träger (2) angepresst, der angeführte räumlich verstellbare Arm (22), **ist dadurch gekennzeichnet, dass** der Ausgleichsmechanismus (24) ferner einen Stabilisator (16) enthält, der mittels eines Bolzenpaares (17) mit dem inneren Gelenk (1) und dem Außengelenk (3) verbunden ist, und der angeführte Stabilisator (16) in dem angeführten Träger (2) ebenfalls enthalten ist.

2. Der räumlich verstellbare Arm nach dem Anspruch 1, **ist dadurch gekennzeichnet, dass** der Höhenausgleichsmechanismus (24) eine Stange (12) enthält, die mittels eines Gleiters (13) im inneren Kopf (18) beweglich eingelagert wird und mittels einer Einlage (21) im Außenkopf (19) beweglich eingelagert wird, wobei an der Stange (12) eine Mutter (10) einstellbar aufgeschraubt wird, die sich auf die an der Stange (12) angebrachte Druckfeder (11) stützt, die sich mit der anderen Seite auf den inneren Kopf (18) stützt, der Gleiter (13) jedoch an die Stange (12) stabil angebracht ist, an der er mit einer Kontermutter (15) gesichert wird, und der Gleiter (13) mittels eines Bolzens (14) mit einem Hacken (9) verbunden ist, der wiederum mittels eines weiteren Bolzens (4) mit einem verstellbaren Stein (5) verbunden ist, der mittels eines Führungsstücks (7) und einer Einstellschraube (6) im inneren Gelenk (1) eingelagert wird.

3. Der räumlich verstellbare Arm nach einem der vorherigen Ansprüche, **ist dadurch gekennzeichnet, dass** er ferner mit dem Arm (23) ein drehbar verbundenes Außengelenk (3) enthält, an dem das hängende Zubehör eingelagert wird.

4. Der räumlich verstellbare Arm nach einem der vorherigen Ansprüche, **ist dadurch gekennzeichnet, dass** an der Oberfläche des inneren Kopfes (18) ein Schutzblech (27) beweglich angebracht wird, das zugleich an der Innenseite der Abdeckung (28) beweglich angebracht wird, die an das innere Gelenk (1) befestigt wird, wobei das Schutzblech (27) am Bolzen (8) eingelagert wird.

5. Der räumlich verstellbare Arm nach einem der vorherigen Ansprüche, **ist dadurch gekennzeichnet, dass** an der Oberfläche des Außenkopfes (19) das Schutzblech (29) beweglich angebracht wird, welches zugleich an der Innenseite der Abdeckung (30) beweglich eingelagert wird, die an das Außengelenk (3) befestigt wird, wobei das Schutzblech (29) am Bolzen (20) eingelagert wird.

6. Der räumlich verstellbare Arm nach einem der vorherigen Ansprüche, **ist dadurch gekennzeichnet, dass** das Außengelenk (3) mit dem Arm (23) mittels eines Bolzens (20) drehbar verbunden ist.

7. Der räumlich verstellbare Arm nach einem der vorherigen Ansprüche, **ist dadurch gekennzeichnet, dass** das innere Gelenk (1) mit dem Arm (23) mittels eines Bolzens (8) drehbar verbunden ist.

## Revendications

1. Le bras transposable dans l'espace (22) du statif de plafond (25) pour l'accrochage et le support des accessoires de suspension (39), qui sont les instruments médicaux, les systèmes, les outils et les appareils avec l'articulation extérieure (3) et qui sont fixées par l'articulation intérieure (1) au système portatif du statif de plafond (25), le bras transposable dans l'espace (22) mentionné contient le bras (23) joint par l'articulation intérieure (1) de manière rotative, composé du support (2) aux bouts duquel sont mis la tête intérieure (18) et la tête extérieure (19, 26), et le mécanisme équilibreur (24) disposé dans le bras (23), tandis que le mécanisme équilibreur (24) est mis dans la tête intérieure (18) et dans la tête extérieure (19), et la tête intérieure (18) et la tête extérieure (19) sont insérées à la presse au support (2), le bras transposable dans l'espace (22) mentionné, **caractérisé par le fait que** le mécanisme équilibreur (24) contient aussi lestabilisateur (16) qui est joint à l'aide du couple des tenons (17) à l'articulation intérieure (1) et à l'articulation extérieure (3), le stabilisateur mentionné (16) est aussi contenu dans le support mentionné (2).

2. Le bras transposable dans l'espace selon la revendication 1, **caractérisé par le fait que** le mécanisme équilibreur réglable en hauteur (24) contient la barre (12) qui est de façon coulissante à l'aide du glisseur (13) mise dans la tête intérieure (18) et de façon coulissante à l'aide de la pièce incérée (21) mise dans la tête extérieure (19), et en même temps sur la barre (12) est mise de façon réglable l'écrou (10) qui est adossé à un ressort de pression (11) mis sur la barre (12) qui est par l'autre côté adossé à la tête intérieure (18), le glisseur (13) est de façon fixe disposé sur la barre (12) où il est bloqué contre le mouvement par le contre-écrou (15), et le glisseur (13) est fixé moyennement le tenon (14) au crochet (9) qui est moyennement d'un autre tenon (4) joint à la pièce transposable (5) qui est à l'aide du guide (7) et le vis réglable (6) placé dans l'articulation intérieure (1).

3. Le bras transposable dans l'espace selon une des revendications précédentes, **caractérisé par le fait que**, puis, il contient l'articulation extérieure (3) de façon pivotante joint au bras (23), et les accessoires de suspension sont accrochées à l'articulation.

4. Le bras transposable dans l'espace selon une des revendications précédentes, **caractérisé par le fait que** sur la surface de la tête intérieure (18) est placée de manière mobile la protection (27) qui est en même temps placée de manière mobile sur le côté intérieur du couvercle (28) qui est fixé à l'articulation intérieure (1), et en même temps la protection (27) est placée au tenon (8).

5. Le bras transposable dans l'espace selon une des revendications précédentes, **caractérisé par le fait que** sur la surface de la tête extérieure (19) est placée de manière mobile la protection (29) qui est en même temps placé de manière mobile sur le côté intérieur du couvercle (30) qui est fixé à l'articulation extérieure (3), et en même temps la protection (29) est placée au tenon (20).

6. Le bras transposable dans l'espace selon une des revendications précédentes, **caractérisé par le fait que** l'articulation extérieure (3) est jointe de manière mobile au bras (23) moyennement du tenon (20).

7. Le bras transposable dans l'espace selon une des revendications précédentes, **caractérisé par le fait que** l'articulation intérieure (1) est jointe de manière mobile au bras (23) moyennement du tenon (8).
